# EUROPEAN PATENT APPLICATION

(11) **EP 4 728 866 A1**
(43) Date of publication of application: **22.04.2026**
(21) Application number: 24823392.6
(22) Date of filing: 12.06.2024
(51) Int. Cl.: A01N 37/46, A01H 1/00, A01H 5/00, A01H 6/46, A01P 5/00, C07K 14/415, C12N 5/10, C12N 15/29, C12N 15/63

(54) **PLANT PARASITIC NEMATODE CONTROLLING AGENT**

(30) Priority: 13.06.2023 JP 2023097058
(71) Applicant: National University Corporation Kumamoto University, Kumamoto 860-8555 (JP)
(72) Inventor: SAWA Shinichiro, Kumamoto-shi, Kumamoto 860-8555 (JP); SUNOHARA Hidehiko, Kumamoto-shi, Kumamoto 860-8555 (JP); SATO Yutaka, Mishima-shi, Shizuoka 411-8540 (JP); DOI Kazuyuki, Nagoya-shi, Aichi 464-8601 (JP)
(74) Representative: J A Kemp LLP
(86) International application number: PCT/JP2024/021268
(87) International publication number: WO 2024/257777

(57) **Abstract**

An object of the present invention is to discover a novel gene that causes a difference in resistance between the plant-parasitic-nematode-resistant rice variety ARC10313 and the plant-parasitic nematode-susceptible rice variety T65 to provide an agent based on the novel gene for controlling a plant-parasitic nematode. Provided is an agent for controlling a plant-parasitic nematode, consisting of a polypeptide or a fragment thereof comprising any amino acid sequence shown in (a) the amino acid sequence of SEQ ID NO: 13, (b) an amino acid sequence in which one or multiple amino acids are deleted, substituted, or added in the amino acid sequence of SEQ ID NO: 13, or (c) an amino acid sequence having 90% or more identity with the amino acid sequence of SEQ ID NO: 13.

## Description

### Technical Field

The present invention relates to: an agent for controlling a plant-parasitic nematode; a plant transformant; and a method for producing the plant transformant.

### Background Art

A plant-parasitic nematode is said to be a parasite on more than 2,000 species of plants, and is known to have an extremely wide host range. Possible host plants include many agriculturally important crops. Damages to the crops have been reported to amount to 150 billion dollars worldwide.

Among the plant-parasitic nematodes, three species of nematodes that are known to be causative of particularly severe agricultural damage are a root-knot nematode, root-lesion nematode, and cyst nematode. For example, *Meloidogyne incognita,* which is one species of root-knot nematode, has a wide host range, parasitizes various plants around the world, such as agricultural crops, and has been reported to cause effects such as reduced yield, reduced quality, growth inhibition, and withering. This is a worldwide problem from the viewpoint of stabilizing food production. Measures against the problem need to be taken urgently.

In general, agricultural damage caused by a plant-parasitic nematode progresses, hidden in the soil, and thus, is difficult to control. Accordingly, spraying a highly toxic chemical (agent for killing a nematode) has been used as an effective control technique against a plant-parasitic nematode. However, a highly toxic chemical substance such as an agent for killing a nematode is problematic in that the substance causes soil contamination. Methyl bromide, which is a main agent for killing a nematode, is deemed to be causative of ozone layer depletion, and has been banned since 2005. Accordingly, there is a demand for an effective nematode control technique that does not rely on an agent for killing a nematode.

The use of a gene resistant to a plant-parasitic nematode is being promoted as a promising control technique that is to replace an agent for killing a nematode. In a tomato, the Mi-1 gene has been found to be a gene resistant to *Meloidogyne incognita* (Non-patent Literature 1). A tomato having the Mi-1 gene introduced thereinto exhibits strong resistance to *Meloidogyne incognita.* Thus, tomatoes having this gene introduced thereinto are widely used in agricultural fields. It has also been reported that lettuce having the tomato-derived Mi-1 gene introduced thereinto acquires resistance to a nematode (Non-Patent Literature 2).

However, the range of plant species for which the Mi-1 gene can be used is limited. No effective nematode-resistant gene is known, particularly for a monocotyledonous plant. In the case of monocotyledonous plants, reports on agricultural damage caused by nematodes to not only major cereals but also bananas, pineapples, and sugarcane have been made one after another. In the case of rice, Meloidogyne graminicola, which is one species of root-knot nematode, has caused severe damage to rice in Asia. It has been reported that, in areas infected with Meloidogyne graminicola, yields are decreased by approximately 70% (Non-patent Literature 3 and 4).

Therefore, there is a demand for a new gene resistant to a plant-parasitic nematode and a new agent based thereon for controlling a plant-parasitic nematode.

### Citation List

### Non-patent Literature

Non-patent Literature 1: Milligan, S. B., et al., 1998, Plant Cell, 10: 1307-1319.
Non-patent Literature 2: Zhang, L.-Y., et al., 2010, Plant Mol Biol Report, 28: 204-211.
Non-patent Literature 3: De Waele, D. and Elsen, A., 2007, Annu. Rev. Phytopathol. 45: 457-485.
Non-patent Literature 4: Bridge, J., Plowright, R. A., and Peng, D., 2005, Plant parasitic nematodes in subtropical and tropical agriculture, pp. 87-130.

### Patent Literature

Patent Literature 1: WO2022/176971

### Summary of Invention

### Technical Problem

An object of the present invention is to discover a novel gene that causes a difference in resistance between the plant-parasitic-nematode-resistant rice variety ARC10313 and the plant-parasitic nematode-susceptible rice variety T65, and to provide an agent for controlling a plant-parasitic nematode, the agent based on the novel gene.

### Solution to Problem

In the past research, the present inventors have focused attention on the *indica* variety Kalo Dhan resistant to *Meloidogyne incognita,* created 128 recombinant inbred lines (RILs) between the *indica* variety and the susceptible *japonica* variety Taichung No. 65 (T65), and performed QTL analysis and gene mapping on the basis of the RILs (Patent Literature 1). As a result, the present inventors have revealed that the Os04g0112100 gene (herein referred to as ROOT KNOT NEMATODE RESISTANCE 1 or RKNR1) is one of the genes that causes a difference in nematode resistance between the two varieties Kalo Dhan and T65.

However, in a comparative study described on the two varieties Kalo Dhan and T65 in Patent Literature 1, the ratio of contribution of the RKNR1 gene to nematode resistance in the RILs produced between the two varieties was only 29.84%. In addition, although the nematode resistance of a transformant obtained by introducing the Kalo Dhan-derived RKNR1 gene into a nematode-susceptible variety is augmented, the nematode resistance did not reach the nematode resistance of Kalo Dhan itself. Accordingly, the RKNR1 alone was not able to explain the difference in nematode resistance between the two varieties, Kalo Dhan and T65.

The present inventors attempted to identify additional resistance genes between the two varieties Kalo Dhan and T65, using the above-mentioned RIL lines, but were unable to map any resistance gene other than the RKNR1 gene in the RIL lines. Hence, a gene that is other than the RKNR1 gene and that causes a difference in nematode resistance between the two varieties Kalo Dhan and T65, has not been identified, and remains unknown to this day.

In view of this, to solve the above-described problems, the present inventors focused attention on ARC10313 that is a resistant variety different from the varieties studied as described above, and have conceived the idea of searching for a new nematode-resistant gene different from the genes studied as described above. Specifically, the inventors have sought a gene that causes a difference in resistance between the resistant variety ARC10313 and the susceptible variety T65, and created a new RIL line between the two varieties ARC10313 and T65. A QTL analysis was performed on this new RIL. Furthermore, gene mapping was also performed, using a chromosome segment substitution line (CSSL). As a result, the inventors have found out the Os06g0621600 gene (herein referred to as ROOT *MELOIDOGYNE INCOGNITA* RESISTANCE 1 or RMIR1) as a new gene associated with the difference in nematode resistance between the two varieties ARC10313 and T65. Furthermore, the inventors introduced the RMIR1 gene of a nematode-resistant variety into a nematode-susceptible variety to produce a transformant, and consequently found out that the transformant acquired nematode resistance. Thus, the inventors have come to complete the present invention. The present invention is based on the above-described findings, and provides the following.

(1) An agent for controlling a plant-parasitic nematode, comprising an RMIR1 polypeptide or a fragment thereof comprising any amino acid sequence shown in the following (a) to (c):
   (a) the amino acid sequence of SEQ ID NO: 13;
   (b) an amino acid sequence in which one or multiple amino acids are deleted, substituted, or added in the amino acid sequence of SEQ ID NO: 13; or
   (c) an amino acid sequence having 90% or more identity with the amino acid sequence of SEQ ID NO: 13.
(2) The agent for controlling a plant-parasitic nematode of (1), further comprising an RKNR1 polypeptide or a fragment thereof comprising any amino acid sequence shown in the following (d) to (f):
   (d) the amino acid sequence of SEQ ID NO: 1;
   (e) an amino acid sequence in which one or multiple amino acids are deleted, substituted, or added in the amino acid sequence of SEQ ID NO: 1; or
   (f) an amino acid sequence having 90% or more identity with the amino acid sequence of SEQ ID NO: 1.
(3) An agent for controlling a plant-parasitic nematode, comprising an RMIR1 polynucleotide encoding the RMIR1 polypeptide or a fragment thereof of (1).
(4) The agent for controlling a plant-parasitic nematode of (3), wherein the RMIR1 polynucleotide comprises any base sequence shown in the following (g) to (j):
   (g) the base sequence of SEQ ID NO: 14;
   (h) a base sequence in which one or multiple bases are deleted, substituted, or added in the base sequence of SEQ ID NO: 14;
   (i) a base sequence having 90% or more identity with the base sequence of SEQ ID NO: 14; or
   (j) a base sequence that hybridizes with a base sequence complementary to the base sequence of SEQ ID NO: 14 in a high-stringent condition.
(5) The agent for controlling a plant-parasitic nematode of (3) or (4), further comprising an RKNR1 polynucleotide encoding the RKNR1 polypeptide or a fragment thereof of (2).
(6) The agent for controlling a plant-parasitic nematode of (5), wherein the RKNR1 polynucleotide comprises any base sequence shown in the following (k) to (n):
   (k) the base sequence of SEQ ID NO: 2;
   (l) a base sequence in which one or multiple bases are deleted, substituted, or added in the base sequence of SEQ ID NO: 2;
   (m) a base sequence having 90% or more identity with the base sequence of SEQ ID NO: 2; or
   (n) a base sequence that hybridizes with a base sequence complementary to the base sequence of SEQ ID NO: 2 in a high-stringent condition.
(7) An agent for controlling a plant-parasitic nematode, comprising an expression vector comprising the RMIR1 polynucleotide of (3) or (4).
(8) The agent for controlling a plant-parasitic nematode of any one of (1) to (7), wherein the plant-parasitic nematode is a root-knot nematode.
(9) The agent for controlling a plant-parasitic nematode of (8), wherein the root-knot nematode is selected from the group consisting of *Meloidogyne graminicola, Meloidogyne incognita, Meloidogyne hapla*, and *Meloidogyne javanica.*
(10) A plant transformant resistant to a plant-parasitic nematode, comprising the agent for controlling a plant-parasitic nematode of (1), (2), or any one of (3) to (9), or a progeny thereof retaining the polynucleotide or the expression vector.
(11) The plant transformant or the progeny thereof of (10), which is a monocotyledonous plant.
(12) The plant transformant or the progeny thereof of (11), wherein the monocotyledonous plant is a Poaceae plant.
(13) The plant transformant or the progeny thereof of (12), wherein the Poaceae plant is selected from the group consisting of rice, wheat, barley, rye, corn, sugarcane, foxtail millet, Proso millet, barnyard millet, and sorghum.
(14) The plant transformant or the progeny thereof of any one of (10) to (13), which is genetically modified.
(15) A method for producing a plant transformant resistant to a plant-parasitic nematode, comprising:
   a step of introducing the expression vector of (7) into a plant; and
   a step of selecting a plant into which the expression vector is introduced.

The present specification encompasses the disclosure of Japanese Patent Application No. 2023-097058 that serves as the basis of the priority of the present application.

### Advantageous Effects of Invention

The present invention can provide an agent for controlling a plant-parasitic nematode, the agent based on the RMIR1 gene that exhibits resistance to a plant-parasitic nematode.

### Brief Description of Drawings

[Figure 1] Figure 1 shows the results of evaluating the resistance of 77 rice varieties to *Meloidogyne incognita.* The graph shows that the lower the evaluation value (EV) is, the higher the resistance to a nematode is. The error bars indicate standard errors.
[Figure 2] Figure 2 shows the results of gene mapping related to resistance to *Meloidogyne incognita.* Figure 2A shows nine SNP markers on chromosome 6 and the evaluation values (EV) with respect to each chromosome segment substitution line (CSSL). For the SNP markers in the Figure, "A" denotes the T65 type, and "B" denotes the ARC10313 type. ** denotes P < 0.01 (the Student's t-test). The error bars indicate standard errors. Figure 2B shows the position of each SNP marker on chromosome 6. The genomic region on which the RMIR1 gene was mapped is denoted by a double-pointed arrow.
[Figure 3] Figure 3 shows the structures of the polypeptides encoded by the RMIR1 genes in ARC10313, N22, Kalo Dhan, T65, and Nipponbare.
[Figure 4] Figure 4 shows the results of evaluating the resistance of the RMIR1 transformant to *Meloidogyne incognita.* ** denotes P < 0.01 (the Student's t-test). The error bars indicate standard errors.
[Figure 5] Figure 5 shows the results of evaluating the resistance of the 77 rice varieties, the RKNR1 transformant, and the RMIR1 transformant to *Meloidogyne incognita.* The error bars indicate standard errors.

### Description of Embodiments

### 1. Agent for controlling plant-parasitic nematode

### 1-1. Overview

A first aspect of the present invention is an agent for controlling a plant-parasitic nematode. An agent for controlling a plant-parasitic nematode of the present invention consists of or comprises a nematode-resistant RMIR1 polypeptide or a fragment thereof, or comprises a polynucleotide encoding any of the polypeptide or the fragment thereof, or an expression vector comprising the polynucleotide. The agent for controlling a plant-parasitic nematode of the present invention has a control effect against a plant-parasitic nematode.

### 1-2. Definitions of terms

Herein, a "plant-parasitic nematode" is not particularly limited, subject to being a nematode that can parasitize a plant. Examples of known plant-parasitic nematodes include root-knot nematodes (*Meloidogyne*), root-lesion nematodes (*Pratylenchus*), cyst nematodes (there are six known genera of cyst nematodes: *Afenestrata, Cactodera*, *Dolichodera*, *Globodera*, *Heterodera*, and *Punctodera*), leaf nematodes (*Aphelenchoides*), and stem nematodes (*Ditylenchus*)*.* A plant-parasitic nematode to be controlled by the present invention is preferably a root-knot nematode.

A root-knot nematode parasitizes a plant root, obtains nutrients from the protoplasm of the plant cells, and forms a knot on the plant root. A root-knot nematode larva molts once inside the egg to become the second stage (J2) larva, which is then hatched. The second stage larva migrates through the soil, invades a tissue near the apical area of a plant, settles near the vascular bundle, ingests nutrients, and then undergoes a second molt to become an imago. The body length of the imago is approximately 0.5 to 1 mm. The female imago excretes an ootheca, and lays approximately 400 to 1500 eggs in the ootheca. Examples of the species belonging to *Meloidogyne* include *Meloidogyne graminicola*, *Meloidogyne javanica*, *Meloidogyne incognita* (herein often referred to as "Mi"), *Meloidogyne hapla*, *Meloidogyne mali*, and *Meloidogyne arenaria.*

Examples of the species belonging to *Pratylenchus* include *Pratylenchus penetrans*, *Pratylenchus coffeae*, *Pratylenchus neglectus*, *Pratylenchus crenatus*, *Pratylenchus vulnus*, and *Pratylenchus loosi.*

Examples of the species belonging to *Heterodera* include *Globodera rostochiensis*, *Heterodera glycines*, and *Heterodera trifolii.*

Examples of the species belonging to *Aphelenchoides* include *Aphelenchoides ritzemabosi, Aphelenchoides fragariae*, *and Aphelenchoides besseyi.*

Examples of the species belonging to *Ditylenchus* include *Dithlenchus destructor and Ditylenchus dipsaci.*

Herein, the plant is not particularly limited, subject to being a plant species that can be parasitized by a plant-parasitic nematode, and may be an angiosperm or a gymnosperm. In addition, angiosperms encompass both dicotyledonous and monocotyledonous plants. Representative examples include plants that are important in agriculture, particularly in the seed and seedling industry and the floriculture industry, for example, crop plants such as grain, flowers, vegetables, and fruits. Specifically, that which falls under the monocotyledonous plant is a species belonging to the family Poaceae (for example, rice, wheat, barley, rye, corn, sugarcane, foxtail millet, Proso millet, barnyard millet, sorghum, or kaoliang), a species belonging to the family Musaceae (for example, banana or *Musa basjoo*), or a species belonging to the family Bromeliaceae (for example, pineapple). That which falls under the dicotyledonous plant is a species belonging to the family Brassicaceae (for example, cabbage, radish, Chinese cabbage, or rapeseed), a species belonging to the family Fabaceae (for example, soybean, peanut, pea, kidney bean, adzuki bean, fava bean, or sweet pea), a species belonging to the family Solanaceae (for example, tomato, eggplant, potato, tobacco, bell pepper, chili pepper, or petunia), a species belonging to the family Convolvulaceae (for example, sweet potato or water spinach), a species belonging to the family Rosaceae (for example, strawberry, rose, apple, pear, peach, loquat, almond, plum, Japanese apricot, or cherry), a species belonging to the family Orchidaceae (for example, Cymbidium, Phalaenopsis, Cattleya, or Dendrobium), a species belonging to the family Liliaceae (for example, lily or tulip), Asparagaceae (for example, hyacinths or muscari), Amaryllidaceae (for example, *Allium,* onion, or garlic), Rutaceae (for example, mandarin orange, orange, grapefruit, lemon, or Citrus junos), a species belonging to the family Vitaceae (for example, grape), a species belonging to the family Asteraceae (for example, lettuce, chrysanthemum, dahlia, marguerite, or sunflower), a species belonging to the family Caryophyllaceae (for example, carnation or baby's breath), or a species belonging to the family Theaceae (for example, sasanqua or *Camellia sinensis*)*.* A root-knot nematode has been reported to parasitize (infect) more than 2,000 species of plants. The species of a host parasitized by a nematode varies depending on the species of the nematode, but nematodes have a wide host range, which includes Solanaceae, Poaceae, Brassicaceae, Fabaceae, Cucurbitaceae, Convolvulaceae, Liliaceae, Asteraceae, Chenopodiaceae, Apiaceae, Araceae, Zingiberaceae, and Malvaceae. Nematodes parasitize various agricultural crops, causing plant diseases. One example of the species of a host plant for a root-knot nematode is tomato, bell pepper, melon, potato, sweet potato, eggplant, carrot, burdock, spinach, Swiss chard, garland chrysanthemum, *Allium,* ginger, pea, kidney bean, cowpea, or rice.

Rice cultivars are classified into Asian rice (*Oryza sativa*) and African rice (*Oryza glaberrima*)*.* Asian rice is classified into two subspecies: *indica and japonica. Japonica* is classified into *Temperate japonica* (shown as "*Temperate japonica*" in Table 1) and *Tropical japonica* (shown as "*Tropical japonica*" in Table 1). Examples of the varieties belonging to *Temperate japonica* include Taichung 65 (T65), Ginbouzu, Nipponbare, Kinmaze, Hinohikari, Yukihikari, Aikoku, Kameji, Kyoutoasahi, Akage, and Dianyu 1. Examples of the varieties belonging to *Tropical japonica* include Ma Sho, Khao Nok, Jaguary, Khau Mac Kho, Padi Perak, Rexmont, Senshou, and Kahei. *Indica* can be classified into *Indica* (shown as "*Indica*" in Table 1) and *Aus* (shown as "*Aus*" in Table 1). Examples of the varieties belonging to *Indica* include Bei Khe, Naba, Puluik Arang, Ryou Suisan Koumai, Jinguoyin, Keiboba, Qingyu, Deng Pao Zhai, Milyang 23, and Karahoushi. Examples of the varieties belonging to *Aus* include Kasalath, Jena 035, Muha, Jhona 2, Nepal 8, Jarjan, Kalo Dhan, Anjana Dhan, Shoni, Surjamukhi, ARC7291, ARC5955, ARC7047, ARC11094, Badari Dhan, Nepal 555, Kaluheenati, DV85, ARC10313, and N22. In addition, Nerica is a hybrid between Asian rice and African rice. Examples of the varieties belonging to Nerica include NERICA 1, NERICA 2, NERICA 4, NERICA 6, NERICA L20, and NERICA L41 (NERICA-related varieties, including NERICA varieties, are shown as "NERICA related" in Table 1). Examples of the hybrids that are not classified into any of the above-described groups (the hybrids are shown as "Admixture" in Table 1) include Davao 1, Asu, IR58, Co 13, Vary Futsi, Shwe Nang Gyi, Pinulupot 1, Local Basmati, Basilanon, Khau Tan Chiem, Tima 1, and Tupa 729. In addition, examples of the varieties the classification of which is unknown (shown as "Unknown" in Table 1) include Basmati 370, IRAT109, LTH, IR24, Kinandang Patong, and Silewah. For a method for classifying rice cultivars, Kojima et al., 2005, Breeding Science, 55, 431-440 and Yonemaru et al., 2014, Plant Cell Physiol., 55 (1), e9 can be referred to.

Rice varieties can be classified into plant-parasitic-nematode-susceptible varieties and plant-parasitic-nematode-resistant varieties. For example, an evaluation value (EV) can be calculated, using the evaluation method described in the Examples herein; a rice variety having an evaluation value equal to or greater than a specific value can be classified as a nematode-susceptible variety; and a rice variety having an evaluation value less than the specific value can be classified as a nematode-resistant variety.

As used herein, the term "plant-parasitic-nematode-resistant" refers to the action of preventing or inhibiting damage to and/or parasitism (infection) in a host plant by a plant-parasitic nematode. Resistance of a plant to a plant-parasitic nematode such as a root-knot nematode can be tested, using a method known to those skilled in the art. For example, a given number of (for example, 200) root-knot nematode J2 larvae are inoculated into a culture soil for a test plant, and the state of infection is evaluated after a given period of time (for example, after 2 months). The evaluation is preferably performed by counting the number of knots and/or the number of root-knot nematode egg masses on the roots of the plant. The number of knots can be counted visually.

As used herein, the term "controlling a plant-parasitic nematode" refers to the action of preventing or inhibiting damage to and/or parasitism (infection) in a host plant by a plant-parasitic nematode.

As used herein, the "RMIR1 (ROOT *MELOIDOGYNE INCOGNITA* RESISTANCE 1) gene" refers to: the Os06g0621600 gene that is on the Nipponbare genome, and associated with resistance to a plant-parasitic nematode in rice; an orthologous gene that is in an arbitrary rice variety, and corresponds to the Os06g0621600 gene; an orthologous gene that is in an arbitrary plant species, and corresponds to the Os06g0621600 gene; or a mutant gene derived from any of the aforementioned genes. The RMIR1 gene is shown as the Os06g0621600 gene in the RAP-db, but is shown as the LOC_Os06g41670.1 gene in the Rice Genome Annotation Project (RGAP). The term "RMIR1 gene" as simply used herein comprises wild-type and mutant RMIR1 genes derived from an arbitrary biological species (the genes are referred to as a "wild-type RMIR1 gene" and a "mutant RMIR1 gene" respectively), and also comprise the below-described L-type RMIR1 gene and S-type RMIR1 gene.

In some cases, there is a difference in the RMIR1 gene sequence among rice varieties. For example, the RMIR1 gene of ARC10313 encodes a protein consisting of 1154 amino acid residues, whereas the RMIR1 gene of T65 encodes a deletion mutant protein consisting of 579 amino acid residues with the C-terminal region deleted. Herein, rice RMIR1 genes are classified into L-type RMIR1 genes and S-type RMIR1 genes, depending on whether the protein has the C-terminal region. That is, the S-type RMIR1 gene encodes a deletion mutant protein in which the C-terminal region is deleted, compared with the L-type RMIR1 gene.

Examples of the L-type RMIR1 gene include the RMIR1 genes of ARC10313, N22, and Kalo Dhan. The base sequences of the RMIR1 genes of ARC10313, N22, and Kalo Dhan are all shown in SEQ ID NO: 14, and are 100% identical.

Examples of the S-type RMIR1 gene include the RMIR1 genes of T65 and Nipponbare. The base sequences of the RMIR1 genes of T65 and Nipponbare are both shown in SEQ ID NO: 16, and are 100% identical.

As used herein, the "RMIR1 polypeptide" refers to a polypeptide encoded by the Os06g0621600 gene that is on the Nipponbare genome, and associated with resistance to a plant-parasitic nematode in rice (i.e., a polypeptide encoded by the RMIR1 gene), an ortholog that is in an arbitrary rice variety, and corresponds to the polypeptide, an ortholog that is in an arbitrary plant species, and corresponds to the polypeptide, or a mutant polypeptide derived from any of the polypeptide and the orthologs. The "RMIR1 polypeptide" as simply used herein comprises wild-type and mutant RMIR1 polypeptides derived from an arbitrary biological species (the polypeptides are referred to as a "wild-type RMIR1 polypeptide" and a "mutant RMIR1 polypeptide" respectively), and also comprises the below-described L-type RMIR1 polypeptide and S-type RMIR1 polypeptide.

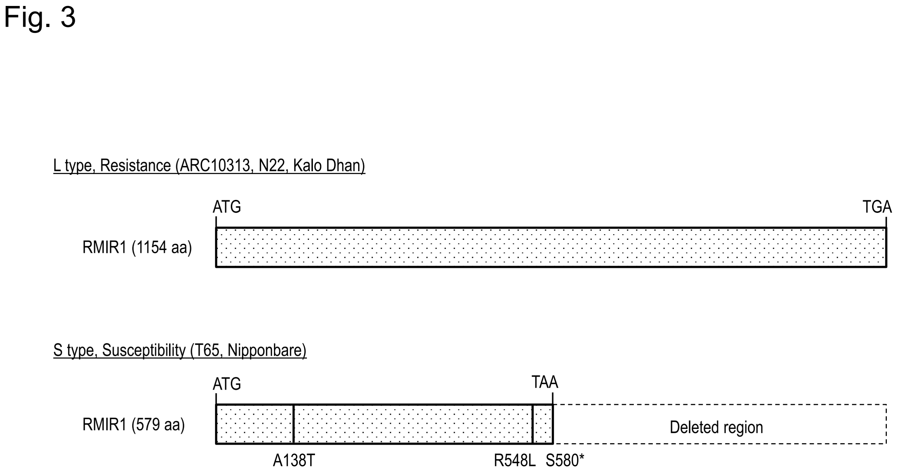

Wild-type rice RMIR1 polypeptides are classified into L-type RMIR1 polypeptides encoded by L-type RMIR1 genes and S-type RMIR1 polypeptides encoded by S-type RMIR1 genes. The L-type RMIR1 polypeptide is an NB-LRR protein comprising a nucleotide-binding domain (NB-ARC domain) and 13 leucine-rich repeat (LRR) domains. Compared with this, in the S-type RMIR1 polypeptide, 11 LRR domains on the C-terminus side of the L-type RMIR1 polypeptide are deleted because the position corresponding to the serine residue at position 580 in the L-type RMIR1 polypeptide is a termination codon. Examples of the amino acid sequence of the L-type RMIR1 polypeptide include SEQ ID NO:13. The amino acid sequences of the RMIR1 polypeptides of ARC 10313, N22, and Kalo Dhan are all shown in SEQ ID NO: 13, and are 100% identical. Examples of the amino acid sequence of the S-type RMIR1 polypeptide include SEQ ID NO:15. The amino acid sequences of the RMIR1 polypeptides T65 and Nipponbare are both shown in SEQ ID NO: 15, and are 100% identical. In the RMIR1 polypeptides of T65 and Nipponbare, 575 amino acid residues on the C-terminus side are deleted, compared with the RMIR1 polypeptides of ARC10313, N22, and Kalo Dhan. Besides, the alanine residue at position 138 is substituted with a threonine residue, and the arginine residue at position 548 is substituted with a leucine residue (Figure 3).

As used herein, the "RKNR1 (ROOT KNOT NEMATODE RESISTANCE 1) gene" refers to: the Os04g0112100 gene that is on the Nipponbare genome, and associated with resistance to a plant-parasitic nematode in rice; an orthologous gene that is in an arbitrary rice variety, and corresponds to the Os04g0112100 gene; an orthologous gene that is in an arbitrary plant species, and corresponds to the Os04g0112100 gene; or a mutant gene derived from any of the aforementioned genes. The "RKNR1 gene" as simply used herein comprises wild-type and mutant RKNR1 genes derived from an arbitrary biological species (the genes are referred to as a "wild-type RKNR1 gene" and a "mutant RKNR1 gene" respectively), and also comprises the below-described L-type RKNR1 gene and S-type RKNR1 gene.

In some cases, there is a difference in the RKNR1 gene sequence among rice varieties. For example, the ORF of the RKNR1 gene is known to be different among rice varieties in whether there is a sequence region extending from the center of the NB-ARC domain to, or to the vicinity of, the C-terminus side of the LRR domain in the RKNR1 amino acid sequence. Herein, rice RKNR1 genes are classified into L-type RKNR1 genes and S-type RKNR1 genes, depending on whether this sequence region is present.

Examples of the L-type RKNR1 gene include: the RKNR1 genes of some varieties (for example, Akage and Ginbouzu) belonging to the *Temperate japonica*; and the RKNR1 genes of the varieties belonging to the *indica*, *Tropical japonica*, *Aus*, and NERICA. Examples of the base sequence of the L-type RKNR1 gene include SEQ ID NO: 2 (N22 and Kalo Dhan), SEQ ID NO: 5 (Naba), SEQ ID NO: 6 (Bei Khe), and SEQ ID NO: 7 (Akage). The base sequence of the RKNR1 gene is 100% identical between N22 and Kalo Dhan, and both are shown in SEQ ID NO:2.

Examples of S-type RKNR1 genes include the RKNR1 genes of some *Temperate japonica* varieties (for example, T65, Nipponbare, Kinmaze, Hinohikari, Yukihikari, Aikoku, Kameji, Kyoutoasahi, and Dianyu 1). Examples of the base sequence of the S-type RKNR1 gene include SEQ ID NO: 3 (T65) and SEQ ID NO: 4 (Nipponbare).

As used herein, the "RKNR1 polypeptide" refers to a polypeptide encoded by the Os04g0112100 gene that is on the Nipponbare genome, and associated with resistance to a plant-parasitic nematode in rice (i.e., a polypeptide encoded by the RKNR1 gene), an ortholog that is in an arbitrary rice variety, and corresponds to the polypeptide, an ortholog that is in an arbitrary plant species, and corresponds to the polypeptide, or a mutant polypeptide derived from any of the polypeptide and the orthologs. The "RKNR1 polypeptide" as simply used herein comprises wild-type and mutant RKNR1 polypeptides derived from an arbitrary biological species (the polypeptides are referred to as a "wild-type RKNR1 polypeptide" and a "mutant RKNR1 polypeptide" respectively), and also comprises the below-described L-type RKNR1 polypeptide and S-type RKNR1 polypeptide.

Wild-type rice RKNR1 polypeptides are classified into L-type RKNR1 polypeptides encoded by L-type RKNR1 genes and S-type RKNR1 polypeptides encoded by S-type RKNR1 genes. In rice varieties other than Bei Khe and Naba, the L-type RKNR1 polypeptide is an NB-LRR protein having a nucleotide-binding domain (NB-ARC domain) and seven leucine-rich repeat (LRR) domains. Compared with this, the S-type RKNR1 polypeptide is the same polypeptide as the L-type RKNR1 polypeptide except that part of the nucleotide-binding domain and most of the LRR domain are deleted. In this regard, the RKNR1 polypeptides of Bei Khe and Naba have a gene sequence in which the 1754 bp region is not deleted, and thus, are classified as the L-type RKNR1 polypeptide, but become a protein having a shorter amino acid length owing to a termination codon generated by a frameshift. Examples of the amino acid sequence of the S-type RKNR1 polypeptide include SEQ ID NO:8 (T65) and SEQ ID NO:9 (Nipponbare). Examples of the amino acid sequence of the L-type RKNR1 polypeptide include SEQ ID NO: 1 (N22 and Kalo Dhan), SEQ ID NO: 10 (Naba), SEQ ID NO: 11 (Bei Khe), and SEQ ID NO: 12 (Akage). The amino acid sequence of the RKNR1 polypeptide is 100% identical between Kalo Dhan and N22, and both are shown in SEQ ID NO: 1.

As used herein, the "whole" of a plant body refers to all regions constituting a living plant. In addition, a "part" of a plant body refers to: a partial region constituting a living plant body, specifically, an organ (for example, a root, stem, leaf, flower, epidermis, or a combination thereof, or pollen, egg cell, or seed); and a tissue consisting of a group of morphologically and/or functionally differentiated cells, or part of the tissue; or a cell.

As used herein, the term "multiple" refers to, for example, 2 to 100, 2 to 90, 2 to 80, 2 to 70, 2 to 60, 2 to 50, 2 to 40, 2 to 30, 2 to 20, 2 to 15, 2 to 10, 2 to 7, 2 to 5, 2 to 4, or 2 to 3. In addition, the "amino acid identity" refers to the ratio (%) of the number of amino acid residues to the number of all the amino acid residues, in which the former residues are identical between the amino acid sequences of two polypeptides in comparison, in which the sequences are aligned by suitably inserting a gap into one or both of the sequences, if desired, in such a manner that the number of identical amino acid residues is the largest. Aligning two amino acid sequences to calculate an amino acid identity can be performed, using a known program such as Blast, FASTA, or ClustalW. The "base identity" is also calculated in the same manner.

As used herein, the "(amino acid) substitution" refers to a substitution within a group of conservative amino acids having similar properties such as in charge, side chain, polarity, and aromaticity among the 20 kinds of amino acids that constitute natural proteins. Examples include substitutions within the group of uncharged polar amino acids having a low-polarity side chain (Gly, Asn, Gln, Ser, Thr, Cys, and Tyr), the group of branched-chain amino acids (Leu, Val, and Ile), the group of neutral amino acids (Gly, Ile, Val, Leu, Ala, Met, and Pro), the group of neutral amino acids having a hydrophilic side chain (Asn, Gln, Thr, Ser, Tyr, and Cys), the group of acidic amino acids (Asp and Glu), the group of basic amino acids (Arg, Lys, and His), and the group of aromatic amino acids (Phe, Tyr, and Trp). An amino acid substitution within these groups is known to be less likely to cause a change in the properties of the polypeptide, and thus, is preferable.

As used herein, the "stringent condition" refers to a condition that is less prone to form a nonspecific hybrid. The term "high-stringent condition" refers to a condition that is still less prone to form, or does not form, a nonspecific hybrid. In general, the lower the salt concentration and the higher the temperature in the reaction condition, the more highly stringent the condition. A post-hybridization washing condition includes, for example, washing with 0.1× SSC and 0.1% SDS at 50°C to 70°C, 55°C to 68°C, or 65°C to 68°C. In addition, other conditions such as probe concentration, probe base length, and hybridization time can be suitably combined to increase the stringency of hybridization.

### 1-3. Constitution

An agent for controlling a plant-parasitic nematode of the present invention comprises, as an active ingredient, (1) a plant-parasitic-nematode-resistant RMIR1 polypeptide or a fragment thereof, (2) a polynucleotide encoding a plant-parasitic-nematode-resistant RMIR1 polypeptide or a fragment thereof, or (3) an expression vector comprising a polynucleotide encoding a plant-parasitic-nematode-resistant RMIR1 polypeptide or a fragment thereof.

### 1-3-1. Active ingredient

### (1) Plant-parasitic-nematode-resistant RMIR1 polypeptide or fragment thereof

In one embodiment, an agent for controlling a plant-parasitic nematode of the present invention consists of or comprises a plant-parasitic-nematode-resistant RMIR1 polypeptide or a fragment thereof.

As used herein, the "plant-parasitic-nematode-resistant RMIR1 polypeptide" refers to an RMIR1 polypeptide that provides a host plant with resistance to a plant-parasitic nematode and/or augments the resistance of the host plant to a plant-parasitic nematode, in which the RMIR1 polypeptide is a wild-type or mutant RMIR1 polypeptide derived from a plant-parasitic-nematode-resistant plant.

Examples of the plant-parasitic-nematode-resistant wild-type RMIR1 polypeptide include wild-type L-type RMIR1 polypeptides. Examples of the wild-type L-type RMIR1 polypeptide include: the wild-type RMIR1 polypeptides of ARC10313, N22, and Kalo Dhan, in which the polypeptides are shown in SEQ ID NO: 13; and the wild-type L-type RMIR1 orthologs of other rice varieties or other plant species.

Examples of the plant-parasitic-nematode-resistant mutant RMIR1 polypeptide include a polypeptide comprising: an amino acid sequence in which one or multiple amino acids are deleted, substituted, or added in the amino acid sequence of any of the described plant-parasitic-nematode-resistant wild-type RMIR1 polypeptides: or an amino acid sequence having 60% or more, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more identity with the amino acid sequence of any of the above-described plant-parasitic-nematode-resistant wild-type RMIR1 polypeptides. Examples include a polypeptide comprising: an amino acid sequence in which one or multiple amino acids are deleted, substituted, or added in the amino acid sequence of SEQ ID NO: 13; or an amino acid sequence having 60% or more, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more identity with the amino acid sequence of SEQ ID NO: 13. The plant-parasitic-nematode-resistant mutant RMIR1 polypeptide preferably has an activity of, or an activity equal to or greater than, 50% or more, 60% or more, 70% or more, 80% or more, or 90% or more of the activity of the plant-parasitic-nematode-resistant wild-type RMIR1 polypeptide.

In one embodiment, the plant-parasitic-nematode-resistant mutant RMIR1 polypeptide consists of a polypeptide or a fragment thereof comprising any of (a) the amino acid sequence of SEQ ID NO: 13, (b) an amino acid sequence in which one or multiple amino acids are deleted, substituted, or added in the amino acid sequence of SEQ ID NO: 13, or (c) an amino acid sequence having 90% or more identity with the amino acid sequence of SEQ ID NO: 13.

As used herein, a "fragment" of a plant-parasitic-nematode-resistant RMIR1 polypeptide refers to a fragment of the above-mentioned plant-parasitic-nematode-resistant RMIR1 polypeptide, the fragment having the activity of providing a host plant with resistance to a plant-parasitic nematode and/or augmenting the resistance of the host plant to a plant-parasitic nematode, for example, a fragment having an activity of, or an activity equal to or greater than, 50% or more, 60% or more, 70% or more, 80% or more, or 90% or more of the activity of the plant-parasitic-nematode-resistant RMIR1 polypeptide. Examples include a polypeptide fragment comprising one, two, or three or more LRR domains in the plant-parasitic-nematode-resistant RMIR1 polypeptide. The amino acid length of a polypeptide constituting this fragment is not particularly limited, but may be, for example, a region of at least 50, 100, 150, 200, 250, 300, 350, 400, 450, 500, 550, 600, 650, 700, 750, 800, 850, 900, 950, or 1000 consecutive amino acids in the plant-parasitic-nematode-resistant RMIR1 polypeptide.

In a further embodiment, the agent for controlling a plant-parasitic nematode further comprises a plant-parasitic-nematode-resistant RKNR1 polypeptide or a fragment thereof in addition to a plant-parasitic-nematode-resistant RMIR1 polypeptide or a fragment thereof.

As used herein, the "plant-parasitic-nematode-resistant RKNR1 polypeptide" refers to an RKNR1 polypeptide that provides a host plant with resistance to a plant-parasitic nematode and/or augments the resistance of the host plant to a plant-parasitic nematode, in which the RKNR1 polypeptide is a wild-type or mutant RKNR1 polypeptide derived from a plant-parasitic-nematode-resistant plant. Patent Literature 1 (WO2022/176971) discloses that the RKNR1 gene is a gene capable of augmenting the resistance of a plant to a plant-parasitic nematode, and also discloses that the gene can inhibit the nematode-attracting activity of the plant, nematode migration into the roots, knot formation, knot maturation, nematode growth in the roots, and/or the derivation of a giant cell in a host plant.

Examples of the plant-parasitic-nematode-resistant wild-type RKNR1 polypeptide include L-type RKNR1 polypeptides other than the RKNR1 polypeptides of Bei Khe, Naba, and Akage. Examples of the L-type RKNR1 polypeptides other than the RKNR1 polypeptides of Bei Khe, Naba, and Akage include: the wild-type RKNR1 polypeptide of N22, shown in SEQ ID NO: 1; the wild-type RKNR1 polypeptide of Kalo Dhan, shown in SEQ ID NO: 1; and wild-type RKNR1 orthologs of other rice varieties or other plant species. Examples include the wild-type RKNR1 polypeptide of Ma Sho, the wild-type RKNR1 polypeptide of Khao Nok, the wild-type RKNR1 polypeptide of Jaguary, the wild-type RKNR1 polypeptide of Khau Mac Kho, the wild-type RKNR1 polypeptide of Padi Perak, the wild-type RKNR1 polypeptide of Rexmont, the wild-type RKNR1 polypeptide of Senshou, the wild-type RKNR1 polypeptide of Kahei, the wild-type RKNR1 polypeptide of Puluik Arang, the wild-type RKNR1 polypeptide of Ryou Suisan Koumai, the wild-type RKNR1 polypeptide of Jinguoyin, the wild-type RKNR1 polypeptide of Keiboba, the wild-type RKNR1 polypeptide of Qingyu, the wild-type RKNR1 polypeptide of Deng Pao Zhai, the wild-type RKNR1 polypeptide of Milyang 23, the wild-type RKNR1 polypeptide of Karahoushi, the wild-type RKNR1 polypeptide of Kasalath, the wild-type RKNR1 polypeptide of Jena 035, the wild-type RKNR1 polypeptide of Muha, the wild-type RKNR1 polypeptide of Jhona 2, the wild-type RKNR1 polypeptide of Nepal 8, the wild-type RKNR1 polypeptide of Jarjan, the wild-type RKNR1 polypeptide of Anjana Dhan, the wild-type RKNR1 polypeptide of Shoni, the wild-type RKNR1 polypeptide of Surjamukhi, the wild-type RKNR1 polypeptide of ARC7291, the wild-type RKNR1 polypeptide of ARC5955, the wild-type RKNR1 polypeptide of ARC7047, the wild-type RKNR1 polypeptide of ARC11094, the wild-type RKNR1 polypeptide of Badari Dhan, the wild-type RKNR1 polypeptide of Nepal 555, the wild-type RKNR1 polypeptide of Kaluheenati, the wild-type RKNR1 polypeptide of DV85, the wild-type RKNR1 polypeptide of ARC10313, the wild-type RKNR1 polypeptide of WAB56-50, the wild-type RKNR1 polypeptide of WAB56-104, the wild-type RKNR1 polypeptide of NERICA 1, the wild-type RKNR1 polypeptide of NERICA 2, the wild-type RKNR1 polypeptide of NERICA 4, the wild-type RKNR1 polypeptide of NERICA 6, the wild-type RKNR1 polypeptide of NERICA L20, the wild-type RKNR1 polypeptide of NERICA L41, the wild-type RKNR1 polypeptide of CG14, the wild-type RKNR1 polypeptide of WK18, the wild-type RKNR1 polypeptide of Davao1, the wild-type RKNR1 polypeptide of Asu, the wild-type RKNR1 polypeptide of IR58, the wild-type RKNR1 polypeptide of Co 13, the wild-type RKNR1 polypeptide of Vary Futsi, the wild-type RKNR1 polypeptide of Shwe Nang Gyi, the wild-type RKNR1 polypeptide of Pinulupot 1, the wild-type RKNR1 polypeptide of Local Basmati, the wild-type RKNR1 polypeptide of Basilanon, the wild-type RKNR1 polypeptide of Khau Tan Chiem, the wild-type RKNR1 polypeptide of Tima 1, the wild-type RKNR1 polypeptide of Tupa 729, the wild-type RKNR1 polypeptide of Basmati 370, the wild-type RKNR1 polypeptide of IRAT109, the wild-type RKNR1 polypeptide of LTH, the wild-type RKNR1 polypeptide of IR24, the wild-type RKNR1 polypeptide of Kinandang Patong, and the wild-type RKNR1 polypeptide of Silewah.

Examples of the plant-parasitic-nematode-resistant mutant RKNR1 polypeptide include a polypeptide comprising: an amino acid sequence in which one or multiple amino acids are deleted, substituted, or added in the amino acid sequence of any of the described plant-parasitic-nematode-resistant wild-type RKNR1 polypeptides: or an amino acid sequence having 60% or more, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more identity with the amino acid sequence of any of the above-described plant-parasitic-nematode-resistant wild-type RKNR1 polypeptides. Examples include a polypeptide comprising: an amino acid sequence in which one or multiple amino acids are deleted, substituted, or added in the amino acid sequence of SEQ ID NO: 1; or an amino acid sequence having 60% or more, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more identity with the amino acid sequence of SEQ ID NO: 1. The plant-parasitic-nematode-resistant mutant RKNR1 polypeptide preferably has an activity of, or an activity equal to or greater than, 50% or more, 60% or more, 70% or more, 80% or more, or 90% or more of the activity of the plant-parasitic-nematode-resistant wild-type RKNR1 polypeptide.

In a further embodiment, the plant-parasitic-nematode-resistant mutant RKNR1 polypeptide is an amino acid residue (for example, Asp) in which position 315 in the amino acid sequence of SEQ ID NO: 12 is other than Gly, an amino acid residue (for example, Glu) in which position 505 in the amino acid sequence of SEQ ID NO: 12 is other than Asp, an amino acid residue (for example, Ala) in which position 745 in the amino acid sequence of SEQ ID NO: 12 is other than Val, and/or an amino acid residue (for example, Leu) in which position 1040 in the amino acid sequence of SEQ ID NO: 12 is other than Gln. The plant-parasitic-nematode-resistant mutant RKNR1 polypeptide is preferably an amino acid residue (for example, Leu) in which position 1040 in the amino acid sequence of SEQ ID NO: 12 is other than Gln.

In a further embodiment, the plant-parasitic-nematode-resistant mutant RKNR1 polypeptide consists of a polypeptide or a fragment thereof comprising any of (a) the amino acid sequence of SEQ ID NO: 1, (b) an amino acid sequence in which one or multiple amino acids are deleted, substituted, or added in the amino acid sequence of SEQ ID NO: 1, or (c) an amino acid sequence having 90% or more identity with the amino acid sequence of SEQ ID NO: 1.

As used herein, a "fragment" of a plant-parasitic-nematode-resistant RKNR1 polypeptide refers to a fragment of the above-mentioned plant-parasitic-nematode-resistant RKNR1 polypeptide, the fragment having the activity of providing a host plant with resistance to a plant-parasitic nematode and/or augmenting the resistance of the host plant to a plant-parasitic nematode, for example, a fragment having an activity of, or an activity equal to or greater than, 50% or more, 60% or more, 70% or more, 80% or more, or 90% or more of the activity of the plant-parasitic-nematode-resistant RKNR1 polypeptide. Examples include a polypeptide fragment comprising an LRR domain in the plant-parasitic-nematode-resistant RKNR1 polypeptide. The amino acid length of a polypeptide constituting this fragment is not particularly limited, but may be, for example, a region of at least 50, 100, 150, 200, 250, 300, 350, 400, 450, 500, 550, 600, 650, 700, 750, 800, 850, 900, 950, or 1000 consecutive amino acids in the plant-parasitic-nematode-resistant RKNR1 polypeptide.

### (2) RMIR1 polynucleotide encoding plant-parasitic-nematode-resistant RMIR1 polypeptide or fragment thereof

In one embodiment, an agent for controlling a plant-parasitic nematode of the present invention comprises or consists of a polynucleotide encoding a plant-parasitic-nematode-resistant RMIR1 polypeptide or a fragment thereof (the polynucleotide is herein referred to as the "RMIR1 polynucleotide").

The RMIR1 polynucleotide encodes the above-described plant-parasitic-nematode-resistant RMIR1 polypeptide or a fragment thereof. The RMIR1 polynucleotide is not particularly limited to any base sequence, subject to being a polynucleotide that encodes a plant-parasitic-nematode-resistant RMIR1 polypeptide or a fragment thereof. For example, the polynucleotide is a polynucleotide encoding the wild-type RMIR1 polypeptide of ARC10313, in which the RMIR1 polypeptide consists of the amino acid sequence of SEQ ID NO: 13 (for example, a polynucleotide which is the wild-type RMIR1 gene of ARC10313, and consists of the base sequence of SEQ ID NO: 14).

In one embodiment, the RMIR1 polynucleotide comprises any of (g) the base sequence of SEQ ID NO: 14, (h) a base sequence in which one or multiple bases are deleted, substituted, or added in the base sequence of SEQ ID NO: 14, (i) a base sequence having 60% or more, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more identity with the base sequence of SEQ ID NO: 14, or (j) a base sequence that hybridizes with a base sequence complementary to the base sequence of SEQ ID NO: 14 in a high-stringent condition.

In a further embodiment, an agent for controlling a plant-parasitic nematode of the present invention further comprises a polynucleotide encoding the plant-parasitic-nematode-resistant RKNR1 polypeptide or a fragment thereof (the polynucleotide is herein referred to as the "RKNR1 polynucleotide"), in addition to a polynucleotide encoding the plant-parasitic-nematode-resistant RMIR1 polypeptide or a fragment thereof.

The RKNR1 polynucleotide encodes the above-described plant-parasitic-nematode-resistant RKNR1 polypeptide or a fragment thereof. The RKNR1 polynucleotide is not particularly limited to any base sequence, subject to being a polynucleotide that encodes a plant-parasitic-nematode-resistant RKNR1 polypeptide or a fragment thereof. Examples include: a polynucleotide encoding the wild-type RKNR1 polypeptide of N22, in which the polypeptide consists of the amino acid sequence of SEQ ID NO: 1 (for example, a polynucleotide that is the wild-type RKNR1 gene of N22, and consists of the base sequence of SEQ ID NO: 2); and a polynucleotide encoding the RKNR1 polypeptide of Kalo Dhan, in which the polypeptide consists of the amino acid sequence of SEQ ID NO: 1 (for example, a polynucleotide that is the wild-type RKNR1 gene of Kalo Dhan, and consists of the base sequence of SEQ ID NO: 2).

In a further embodiment, the RKNR1 polynucleotide comprises any of (k) the base sequence of SEQ ID NO: 2, (l) a base sequence in which one or multiple bases are deleted, substituted, or added in the base sequence of SEQ ID NO: 2, (m) a base sequence having 60% or more, 65% or more, 70% or more, 75% or more, 80% or more, 82% or more, 85% or more, 87% or more, 90% or more, 91% or more, 92% or more, 93% or more, 94% or more, 95% or more, 96% or more, 97% or more, 98% or more, or 99% or more identity with the base sequence of SEQ ID NO: 2, or (n) a base sequence that hybridizes with a base sequence complementary to the base sequence of SEQ ID NO: 2 in a high-stringent condition.

In one embodiment, the base sequence of the RMIR1 polynucleotide and/or the RKNR1 polynucleotide may be a base sequence codon-optimized to match the codon usage in a cell into which the polynucleotide is to be introduced.

The RMIR1 polynucleotide and/or the RKNR1 polynucleotide may be a DNA or an RNA such as an mRNA.

In a case where the RMIR1 polynucleotide and/or the RKNR1 polynucleotide is/are an mRNA, the base sequence thereof can be an mRNA comprising, as a coding region, a base sequence in which thymine (T) in any of the base sequences exemplified above is substituted with uracil (U). In addition to the coding region, the mRNA corresponding to the polynucleotide according to the present invention may comprise a cap structure at the 5' end, a poly A tail at the 3' end, a 5' untranslated region (5' UTR) upstream of the initiation codon, and/or a 3' untranslated region (3' UTR) downstream of the termination codon. The 5' UTR, the 3' UTR, and/or the like may comprise a sequence for regulating the amount of translation from the mRNA.

### (3) Expression vector comprising RMIR1 polynucleotide encoding plant-parasitic-nematode-resistant RMIR1 polypeptide or fragment thereof

In one embodiment, an agent for controlling a plant-parasitic nematode of the present invention comprises or consists of an expression vector comprising the RMIR1 polynucleotide encoding a plant-parasitic-nematode-resistant RMIR1 polypeptide or a fragment thereof.

An expression vector according to the present invention comprises, in an expressible state, the RMIR1 polynucleotide encoding a plant-parasitic-nematode-resistant RMIR1 polypeptide according to the present invention or a fragment thereof. As used herein, the "expressible state" refers to the state in which a gene to be expressed is arranged in a region that is downstream of a promoter and under the control of the promoter.

An expression vector according to the present invention comprises, as essential constituents, a promoter and the polynucleotide described in "(2) RMIR1 polynucleotide encoding plant-parasitic-nematode-resistant RMIR1 polypeptide or fragment thereof".

In a further embodiment, an expression vector according to the present invention further comprises, in an expressible state, the RKNR1 polynucleotide encoding the plant-parasitic-nematode-resistant RKNR1 polypeptide or a fragment thereof, in addition to the RMIR1 polynucleotide encoding the plant-parasitic-nematode-resistant RMIR1 polypeptide or a fragment thereof.

A vector that can be used as an expression vector according to the present invention is, for example, an expression vector prepared utilizing a plasmid or a virus. As used herein, the "expression vector" encompasses a recombinant vector.

In the case of an expression vector prepared utilizing a plasmid (the vector is hereinafter often referred to as a "plasmid expression vector"), that which may be utilized as the plasmid is, for example, but is not limited to, a pPZP-based, pSMA-based, pUC-based, pBR-based, pBluescript-based (Agilent Technologies, Inc.), pTriEXTM-based (TaKaRa Bio Inc.), pBI-based, pRI-based, or pGW-based binary vector.

In the case of an expression vector prepared utilizing a virus (the vector is hereinafter often referred to as a "viral expression vector"), a virus that can be used is a cauliflower mosaic virus (CaMV), bean golden mosaic virus (BGMV), tobacco mosaic virus (TMV), or the like.

In a case where the Agrobacterium method is used, an expression vector suitable for the Agrobacterium method, such as a binary vector, may be used, or a modified vector of the expression vector may be used. Examples of such an expression vector include pBI121, pBIN19, pSMAB704, pCAMBIA, and pGreen.

Various promoters can be used, for example, an overexpression promoter, constitutive promoter, site-specific promoter, time-specific promoter, and/or inducible promoter. Specific examples of the overexpression constitutive promoter that can work in a plant cell include a 35S promoter derived from a cauliflower mosaic virus (CaMV), a Pnos promoter of a nopaline synthase gene derived from a Ti plasmid, a ubiquitin promoter derived from corn, an actin promoter derived from rice, and a PR protein promoter derived from tobacco. Ribulose-bisphosphate carboxylase small subunit (Rubisco ssu) promoters of various plant species, or histone promoters can also be used. Examples of the inducible promoter include inducible promoters such as: a heat shock promoter that can be controlled in accordance with temperature; and a tetracycline-responsive promoter that can be controlled in accordance with whether tetracycline is present.

The expression vector may also comprise a terminator, enhancer, poly A addition signal, 5'-UTR (untranslated region) sequence, intron sequence, ribosome binding sequence, labeling or selection marker gene, multicloning site, nuclease recognition sequence, and/or replication origin. The type of each component is not particularly limited, subject to being capable of achieving the function of the component in a host cell. Any of these components known in the art may be suitably selected, depending on the plant cell or plant host to be introduced.

Examples of the terminator include a terminator of a nopaline synthase (NOS) gene, terminator of an octopine synthase (OCS) gene, CaMV 35S terminator, 3' terminator of Escherichia coli lipopolyprotein lpp, trp operon terminator, amy B terminator, and terminator of an ADH1 gene. The terminator is not particularly limited, subject to having a sequence capable of terminating the transcription of a gene transcribed by the promoter.

Examples of the enhancer include an enhancer region comprising an upstream sequence within the CaMV 35S promoter. The enhancer is not particularly limited, subject to being capable of augmenting the efficiency of expression of a nucleic acid or the like encoding an active peptide.

Examples of the nuclease recognition sequence include: a restriction enzyme recognition sequence; a loxP sequence recognized by a Cre recombinant enzyme; a sequence that is a target of an artificial nuclease such as ZFN and TALEN; and a sequence that is a target of the CRISPR/Cas9 system.

Examples of the replication origin sequence include the SV40 replication origin sequence.

Examples of the selection marker gene include a drug-resistant gene (for example, tetracycline-resistant gene, ampicillin-resistant gene, kanamycin-resistant gene, hygromycin-resistant gene, spectinomycin-resistant gene, chloramphenicol-resistant gene, dihydrofolate reductase gene, or neomycin-resistant gene), fluorescent or luminescent reporter gene (for example, luciferase, β-galactosidase, β-glucuronidase (GUS), or green fluorescent protein (GFP)), and enzyme gene such as neomycin phosphotransferase II (NPT II) or dihydrofolate reductase.

A selection marker gene that can be comprised by an expression vector according to the present invention is a selection marker gene that can select a cell into which the expression vector according to the present invention has been introduced. Specific examples of the selection marker gene include a drug-resistant gene such as an ampicillin-resistant gene, kanamycin-resistant gene, tetracycline-resistant gene, chloramphenicol-resistant gene, neomycin-resistant gene, puromycin-resistant gene, and hygromycin-resistant gene.

A reporter gene that can be comprised by an expression vector according to the present invention is a gene that encodes a reporter capable of identifying a cell into which the expression vector according to the present invention has been introduced. Examples of the reporter gene include: a gene encoding a fluorescent protein such as GFP or RFP; and a luciferase gene.

### 1-3-2. Other ingredients

An agent for controlling a plant-parasitic nematode of the present invention may be composed solely of the active ingredients listed in "1-3-1. Active ingredient", but may comprise another ingredient, if desired.

An agent for controlling a plant-parasitic nematode of the present invention may comprise an agriculturally and pharmaceutically acceptable support. As used herein, the "agriculturally and pharmaceutically acceptable support" refers to a substance that does not substantially affect the activity of the agent for controlling a plant-parasitic nematode of the present invention, and that has no or little harmful effect on the environment, such as soil and water quality, when applied to plant cultivation, or that is not, or only slightly, harmful to animals, in particular humans. Examples of the support include solvents, agents for aiding, agents for shaping, agents for emulsifying, agents for dispersing, and agents for activating a surface.

An agent for controlling a plant-parasitic nematode of the present invention may comprise another ingredient having a pharmacological action, i.e., an agent for killing a nematode, agent for killing an unwanted plant, or fertilizer (for example, urea, ammonium nitrate, or superphosphate) to the extent that the ingredient does not affect the activity of the active ingredient.

### 1-3-3. Dosage form

The dosage form of an agent for controlling a plant-parasitic nematode of the present invention may be in any form as long as the dosage form enables the agent for controlling a plant-parasitic nematode of the present invention to invade into a plant body to which the agent is applied. The dosage form may be, for example, a liquid agent in a liquid state or a solid agent in a solid state. Examples of the liquid agent include: a solution agent in which an active ingredient is suspended in a suitable solution; an oily liquid agent for dispersing; an agent for emulsion; and an agent for suspending. The solid agent is not particularly limited as long as the active ingredient is in condition to act on a plant to which the agent is applied. Examples include powders, dusts, pastes, and gels.

### 1-4. Effects

An agent for controlling a plant-parasitic nematode of the present invention can provide a plant-parasitic-nematode-susceptible plant with resistance to a plant-parasitic nematode. In addition, an agent for controlling a plant-parasitic nematode of the present invention can augment the resistance to a plant-parasitic nematode in a plant resistant to a plant-parasitic nematode.

An agent for controlling a plant-parasitic nematode of the present invention can inhibit, in a plant to which the agent is applied, the following: the nematode-attracting activity; nematode migration into the roots; knot formation; knot maturation; nematode growth in the roots; and/or the derivation of a giant cell in a host plant.

### 2. Plant transformant resistant to plant-parasitic nematode, or progeny of transformant

### 2-1. Overview

A second aspect of the present invention is a plant transformant resistant to a plant-parasitic nematode, or a progeny of the transformant. A plant transformant of the present invention or a progeny thereof comprises the agent for controlling a plant-parasitic nematode according to the first aspect, for example, the polynucleotide or expression vector according to the first aspect, and has resistance to a plant-parasitic nematode such as a root-knot nematode. A plant transformant or a progeny thereof of the present invention achieves inhibition of the following: the nematode-attracting activity; nematode migration into the roots; knot formation; knot maturation; nematode growth in the roots; and/or the derivation of a giant cell in a host plant. Thus, the transformant or a progeny thereof is resistant to a plant-parasitic nematode.

### 2-2. Constitution

As used herein, the "plant transformant" refers to a plant host genetically modified to acquire resistance to a plant-parasitic nematode.

In one embodiment, a plant transformant of the present invention comprises: the RMIR1 polynucleotide encoding a plant-parasitic-nematode-resistant RMIR1 polypeptide or a fragment thereof, the polynucleotide described in the first aspect; or an expression vector comprising the polynucleotide. More specifically, a plant transformant of the present invention is transformed with the RMIR1 polynucleotide encoding a plant-parasitic-nematode-resistant RMIR1 polypeptide or a fragment thereof, the polynucleotide described in the first aspect; or an expression vector comprising the polynucleotide.

In a further embodiment, a plant transformant of the present invention comprises:
the RMIR1 polynucleotide encoding a plant-parasitic-nematode-resistant RMIR1 polypeptide or a fragment thereof, the polynucleotide described in the first aspect; or an expression vector comprising the polynucleotide; and
the RKNR1 polynucleotide encoding a plant-parasitic-nematode-resistant RKNR1 polypeptide or a fragment thereof, the polynucleotide described in the first aspect; or an expression vector comprising the polynucleotide. For example, a plant is transformed with the two kinds of polynucleotides or the expression vector.

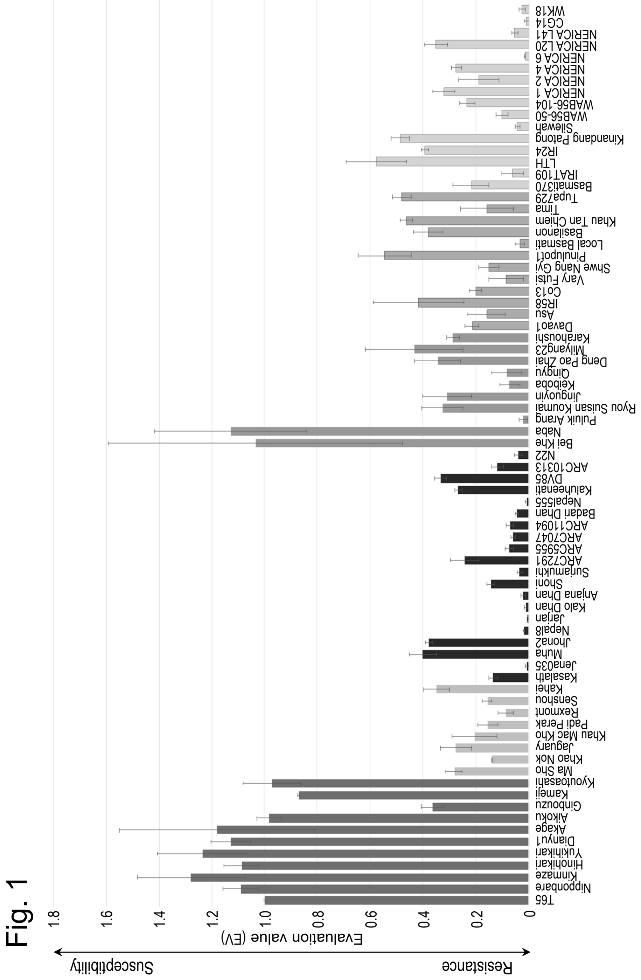

A host plant species to be transformed in the present invention is not limited. The host plant may be a monocotyledonous plant or a dicotyledonous plant, and is particularly preferably a monocotyledonous plant. The monocotyledonous plant may be a species belonging to the family Poaceae (for example, rice, wheat, barley, rye, corn, sugarcane, foxtail millet, Proso millet, barnyard millet, sorghum, or kaoliang), a species belonging to the family Musaceae (for example, banana or Musa basjoo), a species belong to the family Amaryllidaceae (for example, *Allium,* onion, garlic, or leek), or a species belonging to the family Bromeliaceae (for example, pineapple). The host plant is preferably a plant species susceptible to a plant-parasitic nematode or a plant species having weak resistance to a plant-parasitic nematode, and is preferably, for example, a rice variety having the S-type RMIR1 gene and/or the S-type RKNR1 gene, or a plant species not having the L-type RMIR1 gene and/or the L-type RKNR1 gene. Examples of the rice variety susceptible to a plant-parasitic nematode include a rice variety exhibiting an evaluation value of 1.0 or more or a rice variety exhibiting an evaluation value of 0.8 or more in Figure 1.

A plant transformant of the present invention encompasses a clone having the same genetic information. A plant transformant of the present invention also encompasses: a plant body part collected from the first generation of a plant transformant, for example, a plant tissue such as the epidermis, phloem, parenchyma, xylem, or vascular bundle, or a plant organ such as a leaf, petal, stem, root, or seed; or a clone obtained from a plant cell by a plant tissue culture method, cutting, grafting, or layering; a new clone generated from a vegetative propagation organ obtained by asexual reproduction from the first generation of a plant transformant, such as a rhizome, tuberous root, corm, runner, or the like; and an asexual embryo induced by dedifferentiation treatment from the first generation of a plant transformant or a clone derived therefrom.

In one embodiment, a plant transformant of the present invention may be a genetically modified plant.

As used herein, the "progeny" means a host plant that is a descendant generated through sexual reproduction of the first generation of the plant transformant, that holds the RMIR1 polynucleotide encoding a plant-parasitic-nematode-resistant RMIR1 polypeptide or a fragment thereof, or holds an expression vector comprising the polynucleotide, and that is resistant to a plant-parasitic nematode. The progeny is regardless of any generation.

### 2-3. Effects

A plant transformant of the present invention exhibits resistance to a plant-parasitic nematode, in which the plant transformant comprises: the RMIR1 polynucleotide encoding a plant-parasitic-nematode-resistant RMIR1 polypeptide or a fragment thereof; or an expression vector comprising the polynucleotide (the polynucleotide, polypeptide, and vector are hereinafter referred to as the "RMIR1 polypeptide or the like"). In addition, a plant transformant of the present invention comprises: the plant-parasitic-nematode-resistant RMIR1 polypeptide or the like; and the RKNR1 polynucleotide encoding a plant-parasitic-nematode-resistant RKNR1 polypeptide or a fragment thereof; or an expression vector comprising the polynucleotide (the polynucleotide, polypeptide, and vector are hereinafter referred to as the "RKNR1 polypeptide or the like"). The nematode resistance of the plant transformant is further augmented, compared with a transformant comprising only one of the RMIR1 polypeptide or the like and the RKNR1 polypeptide or the like.

### 3. Method for producing plant transformant resistant to plant-parasitic nematode

### 3-1. Overview

A third aspect of the present invention relates to a method for producing a plant transformant resistant to a plant-parasitic nematode. The producing method of the present invention can produce a resistant plant transformant from a plant-parasitic-nematode-susceptible plant.

### 3-2. Method

The method of the present invention for producing a plant transformant resistant to a plant-parasitic nematode comprises an introducing step and a selecting step as essential steps. Each step is specifically described below.

### 3-2-1. Introducing step

In the present aspect, the "introducing step" is a step of introducing, into a host plant, an expression vector comprising the RMIR1 polynucleotide encoding a plant-parasitic-nematode-resistant RMIR1 polypeptide or a fragment thereof

In a further embodiment, the introducing step is a step of introducing, into a host plant, an expression vector comprising the RKNR1 polynucleotide encoding the plant-parasitic-nematode-resistant RKNR1 polypeptide or a fragment thereof, in addition to an expression vector comprising the RMIR1 polynucleotide encoding the plant-parasitic-nematode-resistant RMIR1 polypeptide or a fragment thereof.

The constitution of the expression vector to be introduced in this step is in accordance with the description of "(3) Expression vector comprising RMIR1 polynucleotide encoding plant-parasitic-nematode-resistant RMIR1 polypeptide or fragment thereof' in the first aspect.

As a method for introducing an expression vector, any method known in the art can be used, for example, the Agrobacterium method, PEG-calcium phosphate method, electroporation method, liposome method, particle gun method, or microinjection method. The polynucleotide introduced may be integrated into the genomic DNA of the host, or may exist in the form of an introduced polynucleotide (for example, as it is comprised in an exogenous vector). Furthermore, the polynucleotide introduced may continue to be maintained in the host cell, as in the case where the polynucleotide is integrated into the genomic DNA of the host. Alternatively, the polynucleotide may be maintained transiently.

In a case where the Agrobacterium method is used, a polynucleotide encoding a plant-parasitic-nematode-resistant RMIR1 polypeptide or a fragment thereof is inserted into an expression vector suitable for the Agrobacterium method. The resultant vector is then introduced into a suitable Agrobacterium, for example, Agrobacterium tumefaciens, by the electroporation method or the like. This strain may be inoculated into, and allowed to infect, a plant cell, callus, cotyledonary segment, or the like. Examples of a suitable Agrobacterium strain that can be used include, but are not limited to, GV3101, C58, C58C1Rif(R), EHA101, EHA105, AGL1, and LBA4404.

As a host sample into which the strain is to be introduced, a segment of a plant leaf or the like may be used, or a protoplast may be prepared and used (Christou P, et al., Bio/Technology (1991) 9: 957-962). For example, in the particle gun method, a gene introduction device (for example, PDS-1000 (BIO-RAD Laboratories, Inc.)) can be used in accordance with the manufacturer's instructions to beat, into such a sample, metal particles dredged with an expression vector or DNA construct according to the present invention, thereby introducing the vector or DNA construct into a plant cell and obtaining a plant transformant cell. The method is usually performed under the operating conditions at a pressure of approximately 450 to 2000 psi at a distance of approximately 4 to 12 cm.

### 3-2-2. Selecting step

In the present aspect, the "selecting step" is a step of selecting a plant into which the expression vector has been introduced.

This step may be performed, using a method known in the art, after introducing the expression vector into the host, using the above-described method. For example, a transformant can be selected by utilizing the activity of a protein that is encoded by a selection marker gene or reporter gene in the expression vector.

In one embodiment, a plant cell or a cotyledonary segment into which an expression vector or polynucleotide according to the present invention has been introduced is cultured in a selective medium in accordance with a plant tissue culture method. The surviving callus is cultured in a redifferentiation medium (comprising a suitable concentration of plant hormone (auxin, cytokinin, gibberellin, abscisic acid, ethylene, brassinolide, or the like)), thereby enabling a transformed plant body to be regenerated. In this manner, the transformant can be selected.

### Examples

### <Example 1: Examination of resistance of various rice varieties to Meloidogyne incognita>

### (Purpose)

he purpose is to examine the resistance of various rice varieties to *Meloidogyne incognita* (Mi).

### (Method)

### (1) Rice varieties

The rice varieties evaluated in the present Example were 11 varieties belonging to *Temperate japonica,* 8 varieties belonging to *Tropical japonica,* 20 varieties belonging to *Aus,* 10 varieties belonging to *Indica*, 12 hybrid varieties, 8 varieties belonging to the NERICA-related lineage, and 8 varieties the classification of which is unknown. The above-described varieties were obtained from the National Agriculture and Food Research Organization (NARO) Genbank and Nagoya University.

### (2) Method for evaluating Mi resistance

Mi resistance was evaluated in accordance with the method described in the present inventors' report (Sunohara, H., Kaida, S., and Sawa, S., 2020, Plant Biotechnol., 37, 343-347).

Specifically, rice seeds were immersed in a disinfectant solution (a 1000-fold diluted solution of an agent for bleaching for kitchens, manufactured by Kao Corporation) at 26°C for 3 days (the imbibition period), resulting in killing mold and bacteria and simultaneously inducing germination. After the germination, the seeds were sown in paper pouches (CYG Seed Germination Pouch, Mega International, Inc., USA), two seeds per pouch. The pouches containing the germinated seeds were placed in a dark place at 26°C for 3 days. Next, the 10 pouches were sandwiched between wooden boards, and fixed with spring clamps. The seeds were then grown in a bright place for 12 hours (26°C) and in a dark place for 12 hours (24°C) each day during 8 days. In this regard, the purpose of sandwiching the paper pouches between the wooden boards is to remove excess water from the paper pouches, because such excess water decreases the efficiency of infection of nematodes. After 14 days of imbibition, 2 mL of a solution comprising 400 J2-stage *Meloidogyne incognita* individuals per mL (800 individuals in total) was added along the roots (nematode inoculation). After the inoculation, the pouches sandwiched between the wooden boards were placed horizontally in a dark place for 3 days, and then placed upright in a dark place at 28°C for 12 hours and at 26°C for 12 hours. In addition, the water-saving was maintained during the growing period, and 2 mL of liquid fertilizer described in the literature (Nishiyama H. et al., 2015, Nematol. Res. 45: 45-49) was added to each plant once a week (7, 14, 21, and 28 days after the inoculation).

The number of egg masses was counted 48 days after the imbibition (34 days after the inoculation). The whole root system was immersed in 50 ng/µL erioglaucine for at least 15 minutes to be stained. The number of blue-stained egg masses was counted. After the number of egg masses was counted, the root system was placed in an incubator at 50°C for at least 5 days. The water within the roots was completely removed. Then, the weight of the roots was measured. The number of egg masses per unit dry root weight was calculated from the weight obtained of the dry roots and the number obtained of egg masses. The value standardized on the basis of the T65 value as the reference value was used as the evaluation value (EV) for each variety.

A rice variety the evaluation value of which was 0.6 or greater was classified as the Mi-susceptible variety, and a rice variety the evaluation value of which was less than 0.6 was classified as the Mi-resistant variety.

### (Results)

The respective evaluation values obtained for the varieties are shown in Figure 1 and Table 1 below.

**[Table 1]**

| Table 1. Evaluation values (EV) of various rice varieties | | | | |
|---|---|---|---|---|
| Accession No. | Variety | Place of origin | Classification | Evaluation value (EV) (Mean ± Standard error) |
| - | Taichung65 (T65) | Taiwan | *Temperate japonica* | 1 (reference) |
| WRC 01 | Nipponbare | Japan | *Temperate japonica* | 1.090±0.068 |
| - | Kinmaze | Japan | *Temperate japonica* | 1.278±0.205 |
| - | Hinohikari | Japan | *Temperate japonica* | 1.087±0.065 |
| - | Yukihikari | Japan | *Temperate japonica* | 1.236±0.170 |
| WRC 43 | Dianyu1 | China | *Temperate japonica* | 1.128±0.073 |
| JRC 17 | Akage | Japan | *Temperate japonica* | 1.180±0.371 |
| JRC 26 | Aikoku | Japan | *Temperate japonica* | 0.983±0.045 |
| JRC 27 | Ginbouzu | Japan | *Temperate japonica* | 0.362±0.043 |
| JRC 31 | Kameji | Japan | *Temperate japonica* | 0.869±0.004 |
| JRC 34 | Kyoutoasahi | Japan | *Temperate japonica* | 0.972±0.108 |
| WRC 45 | Ma Sho | Myanmar | *Tropical japonica* | 0.282±0.031 |
| WRC 46 | Khao Nok | Laos | *Tropical japonica* | 0.139±0.002 |
| WRC 47 | Jaguary | Brazil | *Tropical japonica* | 0.275±0.060 |
| WRC 48 | Khau Mac Kho | Vietnam | *Tropical japonica* | 0.204±0.086 |
| WRC 49 | Padi Perak | Indonesia | *Tropical japonica* | 0.154±0.038 |
| WRC 50 | Rexmont | USA | *Tropical japonica* | 0.085±0.029 |
| JRC 04 | Senshou | Japan | *Tropical japonica* | 0.157±0.018 |
| JRC 11 | Kahei | Japan | *Tropical japonica* | 0.348±0.048 |
| WRC 02 | Kasalath | India | *Aus* | 0.133±0.016 |
| WRC 04 | Jena035 | Nepal | *Aus* | 0.006±0.006 |
| WRC 25 | Muha | India | *Aus* | 0.400±0.052 |
| WRC 26 | Jhona2 | India | *Aus* | 0.376±0.015 |
| WRC 27 | Nepal8 | Nepal | *Aus* | 0.018±0.002 |
| WRC 28 | Jarjan | Bhutan | *Aus* | 0.002±0.002 |
| WRC 29 | Kalo Dhan | Nepal | *Aus* | 0.011±0.003 |
| WRC 30 | Anjana Dhan | Nepal | *Aus* | 0.020±0.008 |
| WRC 31 | Shoni | Bangladesh | *Aus* | 0.143±0.014 |
| WRC 33 | Surjamukhi | India | *Aus* | 0.033±0.012 |
| WRC 34 | ARC7291 | India | *Aus* | 0.242±0.054 |
| WRC 35 | ARC5955 | India | *Aus* | 0.073±0.017 |
| WRC 37 | ARC7047 | India | *Aus* | 0.060±0.008 |
| WRC 38 | ARC11094 | India | *Aus* | 0.071±0.015 |
| WRC 39 | Badari Dhan | Nepal | *Aus* | 0.045±0.006 |
| WRC 40 | Nepal555 | India | *Aus* | 0.006±0.006 |
| WRC 41 | Kaluheenati | Sri Lanka | *Aus* | 0.266±0.013 |
| - | DV85 | - | *Aus* | 0.333±0.021 |
| - | ARC10313 | - | *Aus* | 0.117±0.023 |
| - | N22 | - | *Aus* | 0.039±0.016 |
| WRC 03 | Bei Khe | Cambodia | *Indica* | 1.035±0.560 |
| WRC 05 | Naba | India | *Indica* | 1.126±0.289 |
| WRC 06 | Puluik Arang | Indonesia | *Indica* | 0.022±0.015 |
| WRC 09 | Ryou Suisan Koumai | China | *Indica* | 0.326±0.078 |
| WRC 11 | Jinguoyin | China | *Indica* | 0.308±0.092 |
| WRC 17 | Keiboba | China | *Indica* | 0.072±0.038 |
| WRC 18 | Qingyu | Taiwan | *Indica* | 0.083±0.058 |
| WRC 19 | Deng Pao Zhai | China | *Indica* | 0.344±0.085 |
| WRC 57 | Milyang23 | Korea | *Indica* | 0.432±0.185 |
| JRC 44 | Karahoushi | Japan | *Indica* | 0.286±0.024 |
| WRC 07 | Davao1 | Philippines | *Admixture* | 0.213±0.026 |
| WRC 13 | Asu | Bhutan | *Admixture* | 0.158±0.071 |
| WRC 14 | IR58 | Philippines | *Admixture* | 0.416±0.171 |
| WRC 15 | Co13 | India | *Admixture* | 0.199±0.022 |
| WRC 16 | Vary Futsi | Madagascar | *Admixture* | 0.085±0.065 |
| WRC 21 | Shwe Nang Gyi | Myanmar | *Admixture* | 0.151±0.038 |
| WRC 24 | Pinulupot1 | Philippines | *Admixture* | 0.545±0.099 |
| WRC 42 | Local Basmati | India | *Admixture* | 0.032±0.018 |
| WRC 44 | Basilanon | Philippines | *Admixture* | 0.378±0.055 |
| WRC 52 | Khau Tan Chiem | Vietnam | *Admixture* | 0.461±0.025 |
| WRC 53 | Tima | Bhutan | *Admixture* | 0.157±0.099 |
| WRC 55 | Tupa729 | Bangladesh | *Admixture* | 0.479±0.034 |
| - | Basmati370 | India | Unknown | 0.217±0.068 |
| - | IRAT109 | - | Unknown | 0.060±0.042 |
| - | LTH | China | Unknown | 0.576±0.116 |
| - | IR24 | Philippines | Unknown | 0.393±0.015 |
| - | Kinandang Patong | - | Unknown | 0.486±0.034 |
| - | Silewah | - | Unknown | 0.042±0.009 |
| - | WAB56-50 | - | Unknown | 0.100±0.023 |
| - | WAB56-104 | - | Unknown | 0.232±0.029 |
| - | NERICA 1 | - | NERICA related | 0.320±0.041 |
| - | NERICA 2 | - | NERICA related | 0.189±0.076 |
| - | NERICA 4 | - | NERICA related | 0.274±0.020 |
| - | NERICA 6 | - | NERICA related | 0.014±0.003 |
| - | NERICA L20 | - | NERICA related | 0.350±0.043 |
| - | NERICA L41 | - | NERICA related | 0.053±0.012 |
| - | CG14 | - | NERICA related | 0.009±0.009 |
| - | WK18 | - | NERICA related | 0.025±0.012 |

Among *Temperate japonica* varieties, the varieties (T65, Nipponbare, Kinmaze, Hinohikari, Yukihikari, and the like) other than Ginbouzu exhibited Mi susceptibility. On the other hand, among the varieties belonging to the *Tropical japonica, Aus, Indica,* and NERICA, the varieties other than Bei Khe and Naba exhibited Mi resistance.

The *Aus* variety ARC10313 exhibited an evaluation value of approximately 11.7% (EV = 0.117), compared with T65, proving to be one of the varieties having strong resistance to Mi. On the basis of this result, ARC10313 was used as the Mi-resistant variety in the following Examples.

### <Example 2: Mapping and identification of Mi-resistant gene>

### (Purpose)

The purpose is to identify an Mi-resistant gene by performing QTL analysis, using the RIL line, and performing gene mapping, using the CSSL.

### (Method and Results)

### (1) QTL analysis performed, using RILs

Using T65 and ARC10313 as mating parents, 113 recombinant inbred lines (RILs) were created. A QTL (quantitative trait locus) analysis was performed, using 2,144 SNPs. As a result, one of the major QTLs was found on chromosome 6, and named qRMIR1 (qROOT *MELOIDOGYNE INCOGNITA* RESISTANCE 1).

### (2) Gene mapping and identification of the RMIR1 gene, performed using CSSL

In the gene mapping of qRMIR1, no line having, in heterozygosity, a genomic region comprising the qRMIR1 gene was present in the above-described RILs. Accordingly, the gene loci were not able to be narrowed down on the basis of obtaining additional recombinants. However, between the two varieties T65 and ARC10313, a chromosome segment substitution line (CSSL) was distributed from Oryzabase, and accordingly, the gene loci were narrowed down, using the chromosome segment substitution line obtained from Oryzabase. Three CSSLs (TACSSL-42, TACSSL-45, and TACSSL-47) used here have a sequence derived from the resistant variety ARC10313 in a region between 0.596 and 29.229 Mb on chromosome 6, in which the region differs depending on the line. Outside this region, however, the CSSLs have a sequence derived from the susceptible variety T65.

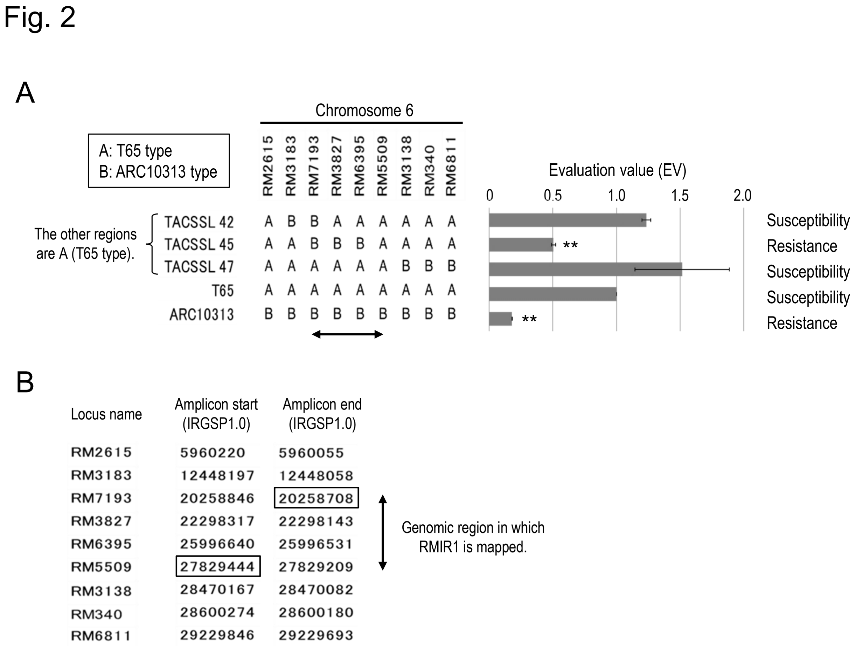

An Mi inoculation test was performed on three CSSLs (TACSSL-42, TACSSL-45, and TACSSL-47) and the parental strains T65 and ARC10313, using the method described in Example 1 to evaluate the resistance. As a result, the evaluation values for TACSSL-42, TACSSL-45, and TACSSL-47 were 1.517 ± 0.035, 0.505 ± 0.015, and 1.236 ± 0.372, respectively (Figure 2A). From these results, the Mi-resistant gene on chromosome 6 was identified in the range of from 20.258 to 27.829 Mb (a region of approximately 7.6 Mb) derived from ARC10313 in TACSSL-45.

Next, the rice database RAP-db (https://rapdb.dna.affrc.go.jp/) was used to search for genes comprised within the range mapped. In this regard, the database was created on the bais of genome information derived from Nipponbare, in which database 449 genes were annotated in the above-described region of approximately 7.6 Mb. From these 449 gene sequences, the Os06g0621600 gene (hereinafter referred to as the ROOT *MELOIDOGYNE INCOGNITA* RESISTANCE 1 gene or RMIR1 gene) was found out as a gene which comprises the NB-ARC domain, and causes a significant difference in the amino acid sequence between a resistant variety and a susceptible variety. The Os06g0621600 gene on the RAP-db corresponds to the LOC_Os06g41670.1 gene in the Rice Genome Annotation Project (RGAP). In the following Examples, the amino acid sequence based on the base sequence of the LOC_Os06g41670.1 gene in the RGAP was regarded as the sequence of the polypeptide encoded by the RMIR1 gene (the polypeptide is hereinafter referred to as the "RMIR1 polypeptide").

The structures of the polypeptides encoded by the RMIR1 gene in the resistant variety ARC10313 and the susceptible variety T65 are shown in Figure 3.

The RMIR1 gene of ARC10313 encodes an L-type RMIR1 polypeptide having a length of 1154 amino acids, and comprising a nucleotide-binding domain (NB-ARC domain) and 13 leucine-rich repeat (LRR) domains. The base sequences of the RMIR1 genes of ARC10313, N22, and Kalo Dhan are all shown in SEQ ID NO: 14, and are 100% identical.

Compared with this, the RMIR1 gene of T65 encodes an S-type RMIR1 polypeptide which has a length of 579 amino acids, in which serine at position 580 is substituted with a termination codon, thus resulting in a deletion of 575 amino acid residues at the C terminus, in which alanine at position 138 is substituted with threonine, and in which arginine at position 548 is substituted with leucine. Accordingly, it was estimated that 11 of the 13 LRR domains were deleted in the RMIR1 gene of T65. In addition, the RMIR1 gene of T65 has, in a region downstream of the termination codon, an insertion sequence that has a length of 141 bases, and is not present in ARC10313. The base sequences of the RMIR1 genes of T65 and Nipponbare are both shown in SEQ ID NO: 16, and are 100% identical.

In this regard, the amino acid identity between the RMIR1 polypeptide (SEQ ID NO: 13) of ARC10313 and the RKNR1 polypeptide (SEQ ID NO: 1) of Kalo Dhan is 62.64%.

### <Example 3: Effect of introduction of RMIR1 gene on Mi resistance trait>

### (Purpose)

The purpose is to create a transformant by introducing, into Nipponbare, a genomic region comprising the RMIR1 gene derived from the nematode-resistant variety Kalo Dhan, and to verify whether the transformant can obtain Mi resistance.

### (Method)

A 12 kb genomic region comprising the Os06g0621600 gene (RMIR1 gene) of wild-type Kalo Dhan was PCR-amplified, and cloned in a vector to create an RMIR1-transformed vector. An RMIR1-transformed vector or a control vector not comprising the RMIR1 genomic region was introduced into the Agrobacterium tumefaciens strain EHA105 by electroporation. Transformation of rice was performed in accordance with the method described in the literature (Toki S., et al, Plant J. 47: 969-976). Specifically, Nipponbare seeds were placed on 2N6 at 28°C for 7 days, immersed in an Agrobacterium suspension for several minutes, and then transferred to a 2N6-AS medium. After the seeds were cocultured at 2°C in a dark place for 3 days, the seeds were washed with sterile water comprising 25 mg/L meropenem (Fujifilm Wako Pure Chemical Corporation) to remove Agrobacterium. The seeds were then cultured on an N6D medium comprising 50 mg/L hygromycin, at 32°C under continuous light for 2 weeks. Subsequently, plantlets were regenerated and collected. In the same manner as in Example 1, an Mi resistance test was made on the RMIR1 transformant obtained by transforming the RMIR1 transformation vector and the vector control strains obtained by transforming the control vectors.

### (Results)

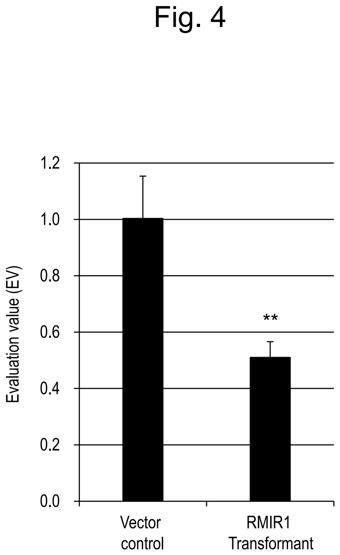

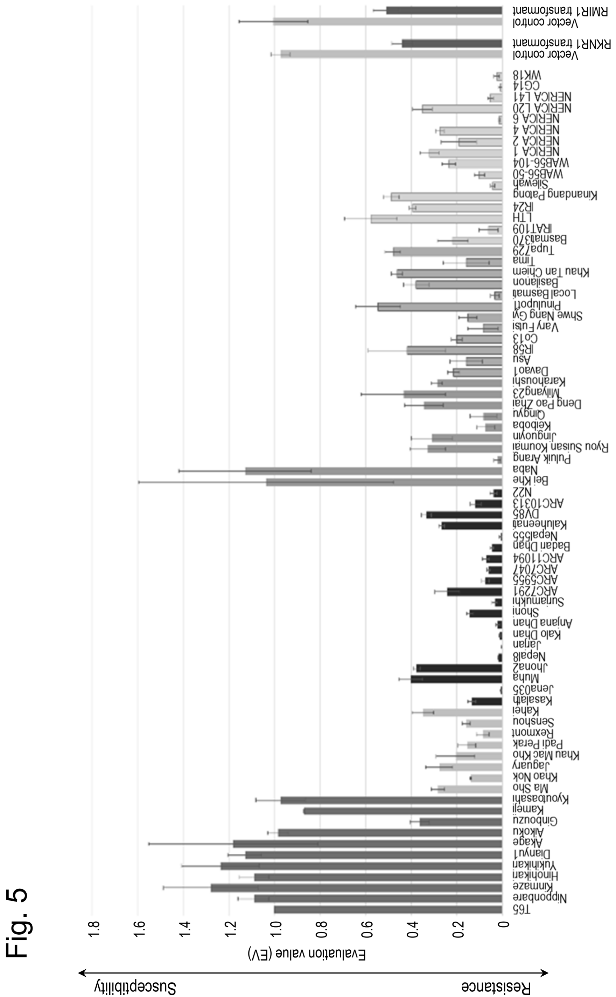

The results are shown in Figure 4 and Figure 5. The evaluation value of the RMIR1 transformant was calculated as a relative value to the average of the evaluation values of the vector controls. The evaluation value of the vector control strains was 1.003 ± 0.150, and the evaluation value of the RMIR1 transformant was 0.510 ± 0.056. A t-test resulted in detecting a significant difference at the 1% level. These results have demonstrated that the RMIR1 gene is a gene responsible for the Mi resistance trait, and that introducing the RMIR1 gene of an Mi-resistant variety into an Mi-susceptible variety provides Mi resistance.

All publications, patents, and patent applications cited herein are incorporated herein by reference in their entirety.

## Claims

1. An agent for controlling a plant-parasitic nematode, comprising an RMIR1 polypeptide or a fragment thereof comprising any amino acid sequence shown in the following (a) to (c):
(a) the amino acid sequence of SEQ ID NO: 13;
(b) an amino acid sequence in which one or multiple amino acids are deleted, substituted, or added in the amino acid sequence of SEQ ID NO: 13; or
(c) an amino acid sequence having 90% or more identity with the amino acid sequence of SEQ ID NO: 13.

2. The agent for controlling a plant-parasitic nematode of claim 1, further comprising an RKNR1 polypeptide or a fragment thereof comprising any amino acid sequence shown in the following (d) to (f):
(d) the amino acid sequence of SEQ ID NO: 1;
(e) an amino acid sequence in which one or multiple amino acids are deleted, substituted, or added in the amino acid sequence of SEQ ID NO: 1; or
(f) an amino acid sequence having 90% or more identity with the amino acid sequence of SEQ ID NO: 1.

3. An agent for controlling a plant-parasitic nematode, comprising an RMIR1 polynucleotide encoding the RMIR1 polypeptide or a fragment thereof of claim 1.

4. The agent for controlling a plant-parasitic nematode of claim 3, wherein the RMIR1 polynucleotide comprises any base sequence shown in the following (g) to (j):
(g) the base sequence of SEQ ID NO: 14;
(h) a base sequence in which one or multiple bases are deleted, substituted, or added in the base sequence of SEQ ID NO: 14;
(i) a base sequence having 90% or more identity with the base sequence of SEQ ID NO: 14; or
(j) a base sequence that hybridizes with a base sequence complementary to the base sequence of SEQ ID NO: 14 in a high-stringent condition.

5. The agent for controlling a plant-parasitic nematode of claim 3, further comprising an RKNR1 polynucleotide encoding the RKNR1 polypeptide or a fragment thereof of claim 2.

6. The agent for controlling a plant-parasitic nematode of claim 5, wherein the RKNR1 polynucleotide comprises any base sequence shown in the following (k) to (n):
(k) the base sequence of SEQ ID NO: 2;
(l) a base sequence in which one or multiple bases are deleted, substituted, or added in the base sequence of SEQ ID NO: 2;
(m) a base sequence having 90% or more identity with the base sequence of SEQ ID NO: 2; or
(n) a base sequence that hybridizes with a base sequence complementary to the base sequence of SEQ ID NO: 2 in a high-stringent condition.

7. An agent for controlling a plant-parasitic nematode, comprising an expression vector comprising the RMIR1 polynucleotide of claim 3.

8. The agent for controlling a plant-parasitic nematode of claim 1, wherein the plant-parasitic nematode is a root-knot nematode.

9. The agent for controlling a plant-parasitic nematode of claim 8, wherein the root-knot nematode is selected from the group consisting of *Meloidogyne graminicola*, *Meloidogyne incognita*, *Meloidogyne hapla*, and *Meloidogyne javanica.*

10. A plant transformant resistant to a plant-parasitic nematode, comprising the agent for controlling a plant-parasitic nematode of any one of claims 3 to 9, or a progeny thereof retaining the polynucleotide or the expression vector.

11. The plant transformant or the progeny thereof of claim 10, which is a monocotyledonous plant.

12. The plant transformant or the progeny thereof of claim 11, wherein the monocotyledonous plant is a Poaceae plant.

13. The plant transformant or the progeny thereof of claim 12, wherein the Poaceae plant is selected from the group consisting of rice, wheat, barley, rye, corn, sugarcane, foxtail millet, Proso millet, barnyard millet, and sorghum.

14. The plant transformant or the progeny thereof of claim 10, which is genetically modified.

15. A method for producing a plant transformant resistant to a plant-parasitic nematode, comprising:
a step of introducing the expression vector of claim 7 into a plant; and
a step of selecting a plant into which the expression vector is introduced.
